# EUROPÄISCHE PATENTANMELDUNG

(11) **EP 2 260 843 A2**
(43) Veröffentlichungstag der Anmeldung: **15.12.2010**
(21) Anmeldenummer: 10181894.6
(22) Anmeldetag: 08.02.2002
(51) Int. Cl.: A61K 31/198, A23L 1/305, A61P 17/00, A61P 29/00, A61K 38/05, A61P 37/08, A61P 37/06, A61P 17/06, A61P 11/06, A61K 38/06, A61P 1/00, C07K 5/06, C07K 5/08

(54) **Alpha-MSH und Derivate zur Induktion von Antigentoleranz**

(30) Priorität: 14.02.2001 DE 10106852
(62) Teilanmeldung aus: 06024288.0
(71) Anmelder: Luger, Thomas, 48147 Münster (DE); Brzoska, Thomas, 48161 Münster (DE); Grabbe, Stephan, 48149 Münster (DE)
(72) Erfinder: Luger, Thomas, 48147, Münster (DE)
(74) Vertreter: Kalhammer, Georg

(57) **Zusammenfassung**

Die vorliegende Erfindung betrifft die Verwendung einer Verbindung, ausgewählt aus αMSH, biologisch aktiven Derivaten davon und biologisch aktiven Fragmenten davon, zur Induktion von Toleranz gegen ein Antigen.

## Beschreibung

Das Tridekapeptid α-Melanocyten-stimulierendes Hormon (αMSH) entsteht aus dem Vorläuferhormon Pro-Opiomelanocortin (POMC). Mehrere biologisch aktive Peptidhormone wie z.B. β-Lipotropin, Adrenocorticotropin (ACTH), β-Endorphin und die Melanotropine (α-, β- und γMSH) leiten sich von dem POMC-Genprodukt ab. Proteolytische Enzyme mit verschiedenen Spezifitäten sind zur Prozessierung dieser Peptide notwendig. Darüber hinaus können posttranslationale Modifikationen wie Acetylierungen stattfinden.

Die Effekte von αMSH und anderen POMC-Peptiden auf die verschiedenen Gewebe werden durch eine Familie spezifischer Rezeptoren vermittelt. Diese Melanocortin (MC)-Rezeptoren gehören zur Gruppe der G-Protein-gekoppelten Rezeptoren. Fünf verschiedene Melanocortinrezeptoren (MC-1 bis MC-5) wurden kloniert. Man nimmt an, daß αMSH ein wichtiges Signal zur Regulation verschiedener Melanocytenfunktionen ist. Beispielsweise sollen die Proliferation, Differenzierung und Cytokinproduktion von Melanocyten durch αMSH beeinflußt werden.

Es wurde auch gezeigt, daß POMC-Genprodukte Immunreaktionen und Entzündungsreaktionen beeinflussen können. Beispielsweise geht man davon aus, daß αMSH mehrere proinflammatorische Cytokine herunterreguliert, während die Produktion des antünflammatorischen Cytokins IL-10 durch αMSH stimuliert wird. Damit hat αMSH eine wichtige Funktion bei der Suppression von Immun- und Entzündungsreaktionen. Mehrere Studien deuten darauf hin, daß die immunmodulatorischen und antiinflammatorischen Effekte von αMSH durch die C-terminale Region von αMSH (Aminosäuren 11-13: Lys-Pro-Val) vermittelt werden, da Verabreichung des C-terminalen Tripeptids hinreichend ist, um diese Effekte zu induzieren (Catania und Lipton, 1993, Endocr. Rev. 14, 564-576; Bhardvaj et al., 1996, J. Immunol. 156, 2517-2521).

WO 88/00833 offenbart die Verwendung des Tripeptids Lys-Pro-Val zur Herstellung eines Medikaments zur Behandlung von Entzündungen. Das C-terminale Tripeptid von αMSH wurde ebenfalls vorgeschlagen als Mittel gegen Haarausfall (FR 2 733 421).

Eine Aufgabe der vorliegenden Erfindung ist es, weitere entzündungshemmende Verbindungen zur Verfügung zu stellen.

Überraschenderweise wurde gefunden, daß das Tripeptid Lys-Pro-Thr antiinflammatorische Eigenschaften aufweist. Wider Erwarten zeigen sogar noch kleinere Verbindungen wie Lys-Pro und Lys vorteilhafte Eigenschaften.

Gegenstand der vorliegenden Erfindung ist daher die Verwendung einer Verbindung der Formel (I) 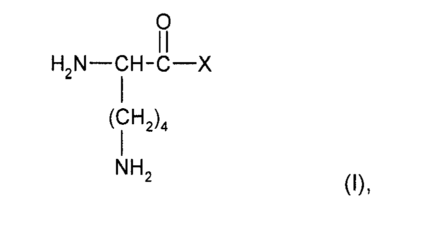 wobei X eine Hydroxylgruppe, eine Aminogruppe, Alkoxy, Pro oder Pro-Thr ist, oder eines pharmazeutisch verträglichen Salzes davon, zur Behandlung und/oder Vorbeugung von entzündlichen Erkrankungen. Der Begriff "entzündliche Erkrankungen umfaßt nicht nur Entzündungen sondern auch solche Erkrankungen, bei denen eine Entzündung beteiligt ist wie beispielsweise Autoimmunerkrankungen oder Transplantatabstoßungen.

Die erfindungsgemäß verwendete Verbindung kann Lysin oder das Dipeptid Lysin-Prolin sein, bevorzugt wird aber das Tripeptid Lysin-Prolin-Threonin (=KPT) verwendet.

Natürlicherweise vorkommende Aminosäuren weisen meist die (L)-Konfiguration auf. Die Aminosäuren der erfindungsgemäß verwendeten Verbindungen können entweder die (L)- oder die (D)-Konfiguration haben. Mögliche Verbindungen der Struktur KPT sind somit
(L)Lys-(D)Pro-(L)Thr,
(L)Lys-(L)Pro-(D)Thr,
(L)Lys-(D)Pro-(D)Thr,
(L)Lys-(L)Pro-(L)Thr,
(D)Lys-(D)Pro-(L)Thr,
(D)Lys-(D)Pro-(D)Thr,
(D) Lys-(L) Pro-(L)Th r,
(D)Lys-(L)Pro-(D)Thr,
am bevorzugtesten ist die Verbindung (L)Lys-(D)Pro-(L)Thr. Die erfindungsgemäß verwendeten Verbindungen können auch Aminosäureaustausche aufweisen, wobei eine der Aminosäuren konservativ getauscht wurde.

Die erfindungsgemäß verwendete Verbindung der Formel (I) kann am N-Terminus und/oder am C-Terminus chemisch modifiziert sein, beispielsweise durch eine Acylgruppe, vorzugsweise eine Acetylgruppe am N-Terminus und/oder eine Amidierung oder Veresterung am C-Terminus. Weitere an sich bekannte Schutzgruppen sind ebenfalls möglich. Die Modifikationen können auch die Aminogruppe in der Seitenkette von Lysin oder die Hydroxylgruppe von Threonin betreffen. Auch auf der Seite der NH₂-Gruppe sind noch andere Modifikationen denkbar, z.B. Verlängerung um ein Glycin, sowie weitere Aminosäurereste bis zur Länge von α-MSH.

Im Rahmen der vorliegenden Anmeldung umfaßt der Begriff "Verbindung der Formel (I)" auch die pharmazeutisch verträglichen Salze der Verbindung.

Die genannten Verbindungen können zur Behandlung aller Arten akuter oder chronischer Entzündungen verwendet werden. Hierzu zählen unter anderem akute und chronische Entzündungen z.B. der Haut, Psoriasis, atopische Dermatitis, allergische Reaktionen aller Art, von Rhinitis über Kontaktallergien bis Asthma und Nahrungsmittelallergien, Autoimmunerkrankungen, Fibrosen und Sklerodermien und Transplantatabstoßung aber auch Gefäßerkrankungen. Vorzugsweise werden die Verbindungen zur Behandlung von entzündlichen Zuständen der Haut verwendet. In diesem Fall ist es vorteilhaft, die Verbindung in Form einer Salbe oder Creme als topische Formulierung zu verabreichen. Üblicherweise ist die Verbindung in einer Salbe oder Creme in einer Konzentration von 1 µM bis 1 mM enthalten, vorzugsweise von 10 µM bis 100 µM. Weiter können in einer derartigen Salbe oder Creme übliche Bestandteile enthalten sein, wie beispielsweise in Braun-Falco et al. (1996) Dermatologie und Venerologie, Springer Verlag, Berlin oder Merk, Bickers (1992) Dermatopharmakologie und Dermatotherapie beschrieben.

In einer bevorzugten Ausführungsform können die Peptide erfindungsgemäß auch für entzündliche Erkrankungen des Darmes verwendet werden. Beispiele für entzündliche Erkrankungen sind neben kurzzeitigen Reizungen des Darmes, die durch leichtere Lebensmittelvergiftungen hervorgerufen werden, auch chronische Erkrankungen des Darmes wie Morbus Crohn oder Colitis Ulcerosa.

In einer anderen bevorzugten Ausführungsform können die Verbindungen erfindungsgemäß zur Behandlung entzündlicher Erkrankungen von Entzündungen verwendet werden die an Stellen des Körpers auftreten, die mit der Außenwelt in Kontakt treten. Hierbei handelt es sich insbesondere um Schleimhaut des Mund- und Magen-DarmTraktes sowie der Lunge.

Die erfindungsgemäß verwendeten Verbindungen sind jedoch auch systemisch wirksam um Entzündungen zu behandeln oder vorzubeugen. Die Verbindung wird dann vorzugsweise intraperitoneal, intravenös oder oral verabreicht. Die Dosis einer Applikation beträgt in der Regel 20 µg bis 10 mg/kg Körpergewicht, bevorzugt 100 µg bis 1 mg/kg Körpergewicht.

Schließlich können die genannten Verbindungen auch in Sprays, beispielsweise zur Inhalation zur Behandlung von Atemwegsentzündungen verwendet werden.

Es ist möglich, daß zur Behandlung mehrere verschiedene Verbindungen der Formel (I) eingesetzt werden. In dieser Ausführungsform werden wenigstens zwei verschiedene Verbindungen der Formel (I) zur Behandlung von Entzündungen verwendet.

Die Verbindungen der Formel (I) können auch zur Herstellung eines Arzneimittels zur Behandlung und/oder Vorbeugung von Entzündungen verwendet werden. Alle oben angegebenen Ausführungsformen sind von dieser Verwendung in analoger Weise mit umfaßt. Die Verbindung wird üblicherweise mit einem pharmazeutisch verträglichen Träger oder Verdünnungsmittel gemischt. An sich bekannte Verfahren zur Herstellung von Arzneimitteln sind in Forth, Henschler, Rummel (1996) Allgemeine und spezielle Pharmakologie und Toxikologie, Urban & Fischer angegeben.

Die Verbindungen der Formel (I) können auch Nahrungsmitteln zugesetzt werden um das allergische Potential bestimmter Nahrungsmittelbestandteile zu reduzieren. Die Erfindung betrifft daher auch die Verwendung einer Verbindung der Formel (I) als Nahrungsmittelzusatz. Die Konzentration in Nahrungsmitteln kann dann 1 µM bis 1 mM betragen.

Erfindungsgemäß ist es auch möglich, eine Verbindung der Formel (I) als nichtpharmazeutischen Zusatz in Kosmetika zu verwenden. Beispielsweise können Cremes bei gereizter Haut oder nach dem Sonnenbad eingesetzt werden, die eine Verbindung der Formel (I) enthalten.

Überraschenderweise wurde von den Erfindern ebenfalls gefunden, daß die Behandlung dendritischer Zellen in vitro mit einem Hapten und αMSH und anschließende Injektion der Zellen in Versuchstiere zur Entstehung Hapten-spezifischer Toleranz und zur Unterdrückung der CHS-Reaktion ("contact hypersensitivity reaction") führt. Daher ist ein weiterer Gegenstand der vorliegenden Erfindung ein Verfahren zur in vitro-Herstellung von Zellen, die Toleranz gegen ein Antigen verleihen können, das umfaßt, daß man Antigen-präsentierende Zellen bereitstellt, die Zellen mit αMSH oder einem biologisch aktiven Derivat oder Fragment davon in Kontakt bringt und die Zellen mit dem Antigen in Kontakt bringt, wobei die letzten beiden Schritte in beliebiger Reihenfolge oder gleichzeitig durchgeführt werden können.

Es gibt verschiedene Arten Antigen-präsentierender Zellen. Bevorzugt gemäß der vorliegenden Erfindung sind dendritische Zellen oder Langerhans-Zellen. Es ist nicht erforderlich, daß die Antigen-präsentierenden Zellen in einer Präparation vorliegen, die frei von anderen Bestandteilen oder Zellen ist. Die Antigen-präsentierenden Zellen können auch im Gemisch mit anderen Zellen zur Verfügung gestellt werden. Beispielsweise werden bevorzugt epidermale Zellen zur Verfügung gestellt, in denen als Antigen-präsentierende Zellen Langerhans-Zellen enthalten sind. Es können auch dendritische Zellen aus Knochenmark isoliert werden oder dendritische Zellen durch an sich bekannte in vitro-Kultur aus Vorläuferzellen wie z.B. PBMC hergestellt werden. Verfahren zur Bereitstellung Antigen-präsentierender Zellen sind beispielsweise in Labeur et al. J. of Immunol. 162(1):168-175 (1999) beschrieben.

Die Zellen werden dann in vitro mit αMSH oder einem biologisch aktiven Derivat oder Fragment davon in Kontakt gebracht werden. Biologische aktive Derivate oder Fragmente von αMSH sind beispielsweise chemische Modifikationen von αMSH, Fragmente von αMSH, die Lys, Lys-Pro, Lys-Pro-Val oder Lys-Pro-Thr umfassen, oder Verbindungen, die eine der genannten Substanzen umfassen. Es sind verschiedenste Abwandlungen denkbar, solange die biologische Aktivität von αMSH - die Fähigkeit zur Toleranzinduktion - im wesentlichen erhalten ist. Übliche Konzentrationen von αMSH oder den genannten Derivaten beim Inkontaktbringen mit den Zellen sind 10⁻⁶ M bis 10⁻¹⁵ M, vorzugsweise 10⁻⁸ M bis 10⁻¹² M.

Nach Inkontaktbringen der Zellen mit αMSH oder einem biologisch aktiven Derivat oder Fragment davon oder vorher oder gleichzeitig werden die Zellen in vitro mit dem Antigen, gegen das Toleranz induziert werden soll, in Kontakt gebracht. Das Antigen kann dabei ein Protein sein, gegen das die Gefahr einer allergischen Reaktion besteht. Wenn beispielsweise bekannt ist, gegen welches Hapten des Antigens sich die Immunreaktion richtet, können die Zellen auch nur mit dem speziellen Hapten in Kontakt gebracht werden. Dabei kann es sich beispielsweise um Peptide in der Länge von 7 bis 20 Aminosäuren, vorzugsweise von 7 bis 15 Aminosäuren handeln.

Die Antigen-präsentierenden Zellen können nach den genannten Schritten gewaschen werden und mit einem pharmazeutisch verträglichen Trägerstoff oder Verdünnungsmittel gemischt werden. Die Zellen können dann in einen Patienten oder in ein Säugetier eingebracht werden, woraufhin Toleranz gegen das verwendete Hapten bzw. Antigen entsteht.

Ein weiterer Aspekt der Erfindung ist die Verwendung von αMSH oder einem biologisch aktiven Derivat oder Fragment davon zur Herstellung eines Arzneimittels zur Induktion von Toleranz gegen ein Antigen. Vorzugsweise enthält das hergestellte Arzneimittel Zellen, die durch das oben beschriebene Verfahren zur in vitro-Herstellung von Zellen, die Toleranz verleihen können, erhältlich sind.

Das Peptid Lys-Pro-Thr verhindert die Aktivierung des Transkriptionsfaktors NF-κB durch TNFα, IL-1 oder LPS in Endothelzellen und in Keratinocyten. In der Folge kommt es zu einer verminderten Expression von Zelladhäsionsmolekülen (Endothelzellen) und Chemokinen (Keratinocyten). Die Erfinder konnten auch zeigen, daß beispielsweise das Peptid KPT das Auftreten von Kontaktallergien (Contact Hypersensitivity Reactions, CHS Reactions) verhindert und eine Allergen-spezifische, langanhaltende Toleranz induziert. Bei den CHS-Reaktionen sind zwei Abschnitte zur unterscheiden: Ein Erstkontakt (Induktionsphase) mit einem Antigen legt den Grundstein für die spätere CHS-Reaktion, ein weiterer Kontakt mit dem Antigen führt zum Auftreten der Reaktion (Kontaktekzem, also Schwellung, Juckreiz, etc.). Die erfindungsgemäß verwendeten Verbindungen können vor beiden Abschnitten eingesetzt werden, bei Einsatz (Injektion oder topische Applikation) vor dem Erstkontakt kommt es zur Unterdrückung der CHS und zur Toleranzinduktion, bei Einsatz zur Zeit der Auslösung des Kontaktekzems verhindern die Verbindungen das Auftreten des Ekzems. In allen diesen Anwendungen kommt es zu einer weitgehend kompletten Inhibition der allergischen Reaktion.

Es wurde ebenfalls gefunden, daß Lys-Pro-Thr die Expression costimulatorischer Moleküle auf dendritischen Zellen reduziert. Dies ist höchstwahrscheinlich ein Teil des Mechanismus bei der Unterdrückung der CHS und der Toleranzinduktion. Gleichzeitig erhöhen die Verbindungen die Sekretion des antiinflammatorischen IL-10 durch Monocyten. Dieser Effekt ist ebenfalls Teil des Mechanismus im Rahmen der allergischen Kontaktekzeme.

Ohne in irgendeiner Art und Weise an eine Theorie gebunden sein zu wollen, könnten die erfindungsgemäßen Verbindungen an β-adrenerge Rezeptoren binden. Weiterhin kann davon ausgegangen werden, daß die erfindungsgemäß eingesetzten Peptide zur Bindung an den IL-1 Rezeptor vom Typ I befähigt sind. Nicht ausgeschlossen werden kann auch, daß die erfindungsgemäßen Peptide auch noch an weitere Rezeptoren, wie beispielsweise den κ-Opioid-Rezeptor binden. Ausgehend von dieser Annahme wird vermutet, daß die erfindungsgemäßen Peptide an mehrere Rezeptoren binden können, die nach Aktivierung durch ihre Original-Liganden alle pro-inflammatorisch in das Entzündungsgeschehen eingreifen würden. Durch die Bindung der erfindungsgemäßen Peptide an diese Rezeptoren wird die Bindung der Original-Liganden an diese Rezeptoren verhindert und dadurch wird die Induktion der pro-inflammatorischen Effekte verhindert. Andererseits aktiviert die Bindung der erfindungsgemäßen Peptide an die Rezeptoren ihrer Ausgangssubstanzen (αMSH) diese Rezeptoren und induziert somit eine weitere Komponente des Wirkmechanismus, der insgesamt antientzündlich ist.
Figur 1 zeigt, daß die intravenöse Injektion von αMSH, KPV oder KPT die Sensibilisierungsphase von CHS unterdrückt.
Figur 2 zeigt, daß die intravenöse Applikation von αMSH, KPV oder KPT Toleranz induzieren kann.
Figur 3 illustriert die IL-10-Sekretion durch humane PBL 24 Stunden nach Behandlung mit αMSH, KPV oder KPT.
Figur 4 zeigt die IL-10-Sekretion durch humane PBL 48 Stunden nach Behandlung mit αMSH, KPV oder KPT.
Figur 5 zeigt, daß THP-1-Zellen Rezeptoren für αMSH exprimieren.
Die Figuren 6a bis d, 7a bis d und 8a bis d zeigen, daß in einem Kompetitionsassay unmarkiertes αMSH, KPV oder KPT Biotin-markiertes αMSH von Bindungsstellen auf THP-1-Zellen verdrängen können.
Figur 9a zeigt die Expression von "Cell Adhesion Molecules" (CAMS) auf der Oberfläche von HMEC-1-Zellen 24 Stunden nach Behandlung durch TNFα + αMSH oder TNFα + KPT.
Figur 9b zeigt die Adhäsion von Lymphocyten an HDMEC (Chromium Release Assay). A: Molt4 T-Lymphocyten; B: JY B-Lymphocyten.
Figur 10 zeigt den Einfluß von αMSH, KP, KPV oder KPT auf die NF-κB-Aktivierung in LPS-behandelten HMEC-1-Zellen.
Figur 11a zeigt, daß die Anzahl E-Selektin-exprimierender Gefäße in Gewebeschnitten durch αMSH-Behandlung erniedrigt wird.

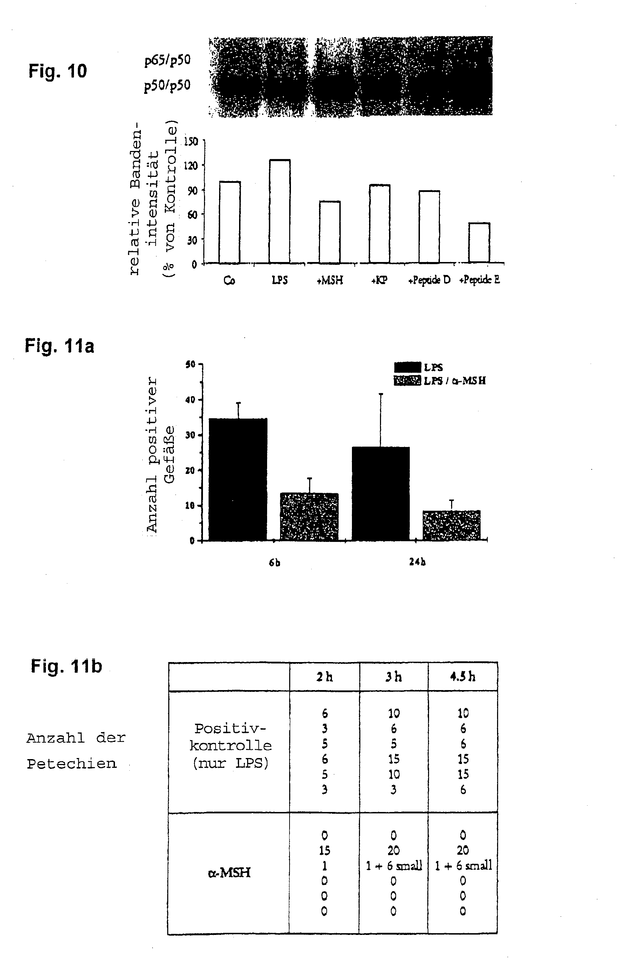

Figur 11 b zeigt, daß die Anzahl petechialer Läsionen an den Ohren LPS-behandelter Mäuse durch αMSH-Behandlung reduziert wird.

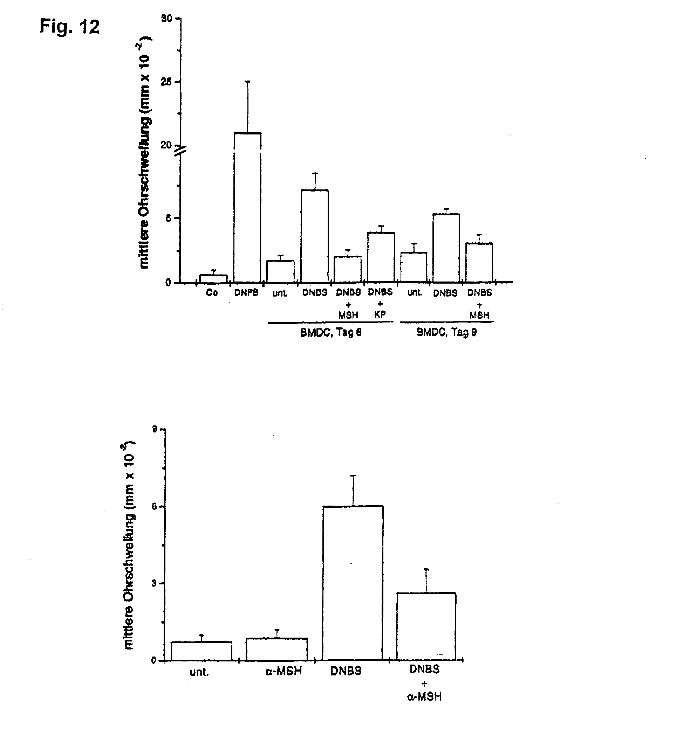

Figur 12 zeigt, daß durch in vitro-Behandlung von BMDC mit αMSH oder KP CHS unterdrückt und Toleranz induziert werden kann.

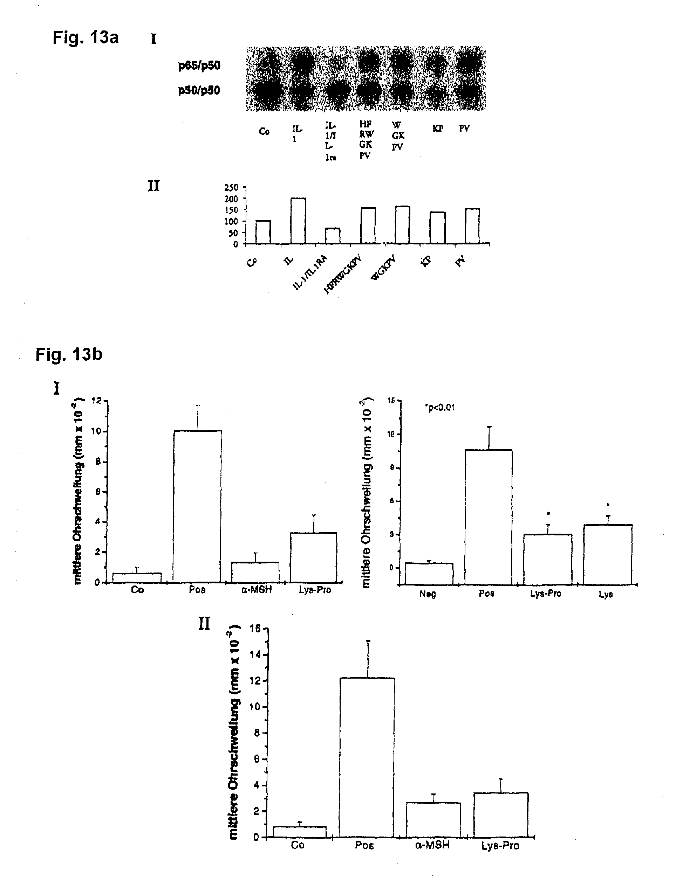

Figur 13a zeigt, daß in einem NF-κB "Band Shift Assay" die Intensität der NF-κB-p65/p50-Heterodimerbande durch verschiedene von αMSH abgeleitete Peptide erniedrigt wird.
Figur 13b zeigt den Effekt von αMSH, KP oder K auf die CHS-Reaktion und den Effekt von αMSH oder KP auf Toleranzinduktion in BalbC-Mäusen.

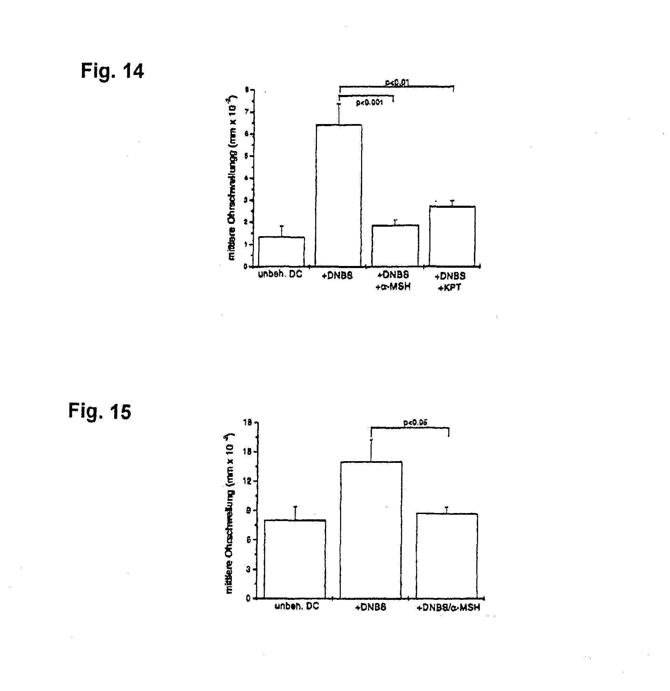

Figur 14 zeigt die CHS-Unterdrückung durch T-Zellen, die in vitro mit Antigen-beladenen und αMSH bzw. Derivat-behandelten DC kontaktiert wurden.
Figur 15 zeigt die Toleranz-Induktion durch T-Zellen, die in vitro mit Antigen-beladenen und αMSH DC kontaktiert wurden.

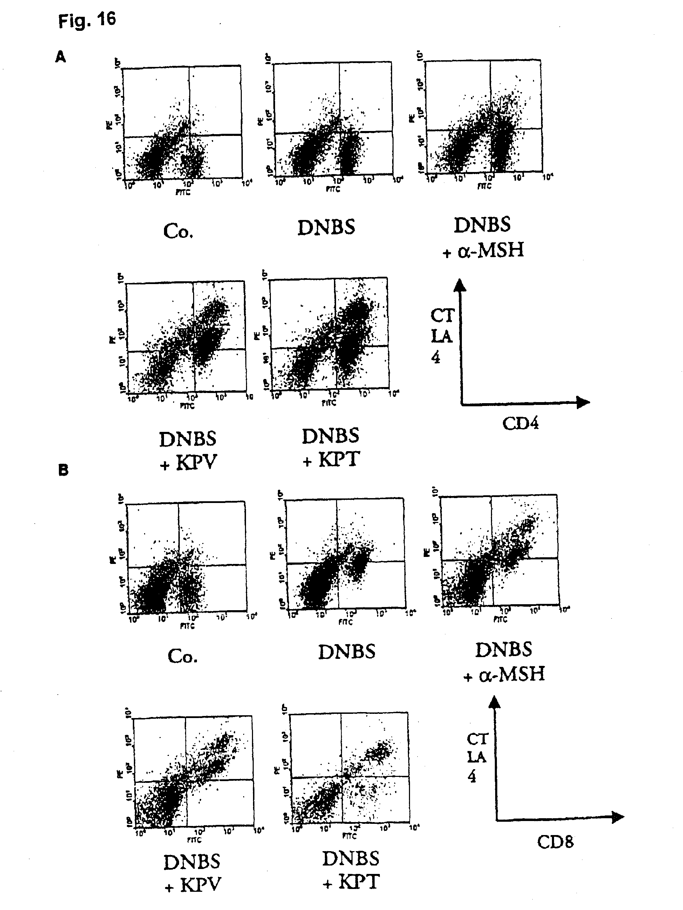

Figur 16 zeigt die Aufregulation von CTLA-4 auf T-Zellen nach Kontakt mit Antigen-beladenen und αMSH bzw. Derivat-behandelten DC. A: CD4 positive T-Zellen; B: CD8 positive T-Zellen.

Die nachfolgenden Beispiele sollen die Erfindung näher erläutern.

### Beispiel 1

### Mäuse:

7 bis 10 Wochen alte, weibliche Balb/C-Mäuse wurden von Charles River (Sulzfeld, Deutschland) erhalten und gemäß Bundesbestimmungen gehalten.

### Verabreichung von αMSH oder KPV oder KPT oder KP:

αMSH und die Peptide wurden als Aliquots bei -20°C bis zur Verwendung aufbewahrt. Vor der Injektion wurde die jeweilige Verbindung in PBS, 0,1% Mausserum gelöst und bis zur i.v.-Injektion in die Schwanzvene der Mäuse auf Eis gelagert. 5 µg αMSH oder 1,5 µg Peptid (KP: 50 µg) pro Maus wurden 2 Stunden vor der Sensibilisierung injiziert.

### Bestimmung von CHS und Toleranz:

Die Mäuse wurden durch Verstreichen von 75 µl 0,5% DNFB in Aceton-Olivenöl (4:1) auf dem rasierten Bauch naiver Mäuse sensibilisiert. Zur Auslösung der CHS wurden die Ohren der Mäuse mit 10 µl 0,3% DNFB auf beiden Seiten eines Ohres bestrichen. CHS wurde bestimmt durch den Grad der Ohrschwellung des Hapten-exponierten Ohres verglichen mit dem des anderen, kontrollbehandelten Ohres und mit einem federgespannten Greifzirkel 24 Stunden nach Hapten-Exposition gemessen. Mäuse, deren Ohren ohne vorherige Sensibilisierung Hapten-exponiert wurden, dienten als Negativkontrolle. Um zu bestimmen, ob die Injektion von αMSH oder Peptiden vor der Haptenapplikation zur Toleranzinduktion führt, wurden Mäuse i.v. mit αMSH oder Peptiden 2 Stunden vor der Sensibilisierung injiziert (Abdomen) oder exponiert (linkes Ohr) wie beschrieben. Zur Bestätigung der αMSH-induzierten Suppression von CHS wurden Mäuse 7 Tage nach der Sensibilisierung an einem Ohr Hapten-exponiert und die Ohrschwellungsantwort 24 bis 36 Stunden später bestimmt. 14 Tage später wurden dieselben Mäuse nochmals am rasierten Rücken sensibilisiert (jetzt in Abwesenheit von exogenem αMSH) und auf ihre Fähigkeit hin untersucht, eine CHS-Antwort durch eine zweite Haptenexposition am rechten Ohr eine Woche später zu induzieren.

In einigen Experimenten wurden topische Zubereitungen von αMSH benutzt. In diesen Experimenten wurden die Mäuse am Ort der Sensibilisierung (Abdomen) unmittelbar vor oder 3 Stunden oder 24 Stunden vor der Sensibilisierung bestrichen.

### Ergebnis:

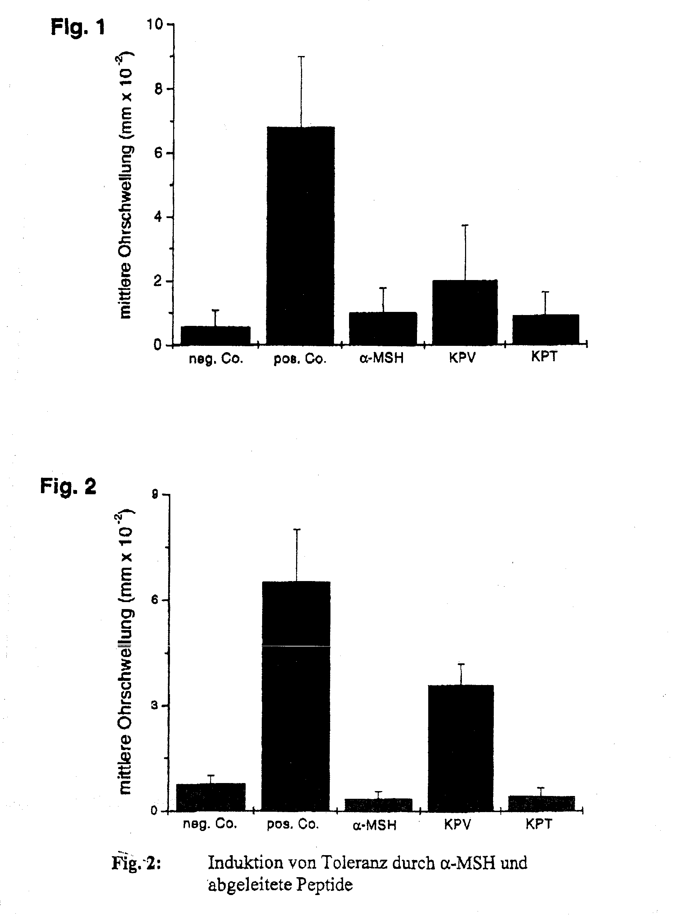

I.v.-Injektion von αMSH sowie von KPV oder KPT oder KP inhibiert die Fähigkeit der Mäuse, eine CHS-Antwort auf eine 7 Tage später erfolgte DNFB-Exposition zu induzieren. Diese Mäuse entwickeln somit keine DNFB-spezifische Sensibilisierung. KPT unterdrückte die CHS-Antwort am effektivsten (siehe Figur 1 und 13b).

Um zwischen temporärer Immunsuppression und spezifischer immunologischer Toleranz zu unterscheiden wurden Mäuse ein zweites Mal sensibilisiert und Hapten-exponiert. Mäuse, die vor der ersten Sensibilisierung mit αMSH oder KPV oder KPT injiziert worden waren, konnten nicht einmal durch Applikation einer zweiten sensibilisierenden Haptendosis sensibilisiert werden, was darauf hindeutet, daß diese Mäuse gegen DNFB Toleranz entwickelt haben. KPV zeigte einen schwachen Effekt, wohingegen αMSH und KPT und KP die Ohrschwellungsantwort sehr stark hemmten (siehe Figur 2 und 13b).

### Beispiel 2

### Material und Methoden:

Mononukleäre Zellen (PBMC) wurden von "Human Buffy Coats" durch Ficoll-Hypaque-Dichtegradientenzentrifugation getrennt. Zellen (1 x 10⁶ pro ml), kultiviert in RPMI 1640 mit Antibiotika und 10% FCS wurden entweder nicht behandelt oder mit αMSH oder den Peptiden KPV oder KPT mit oder ohne IL-1β (10 U/ml) stimuliert. Die Überstände der PBMC-Kulturen wurden nach 24 oder 48 Stunden Inkubation gesammelt und bei -20°C bis zur weiteren Verwendung aufbewahrt. Zur Detektion von IL-10 wurde ein kommerziell erhältlicher ELISA eingesetzt.

### Ergebnisse:

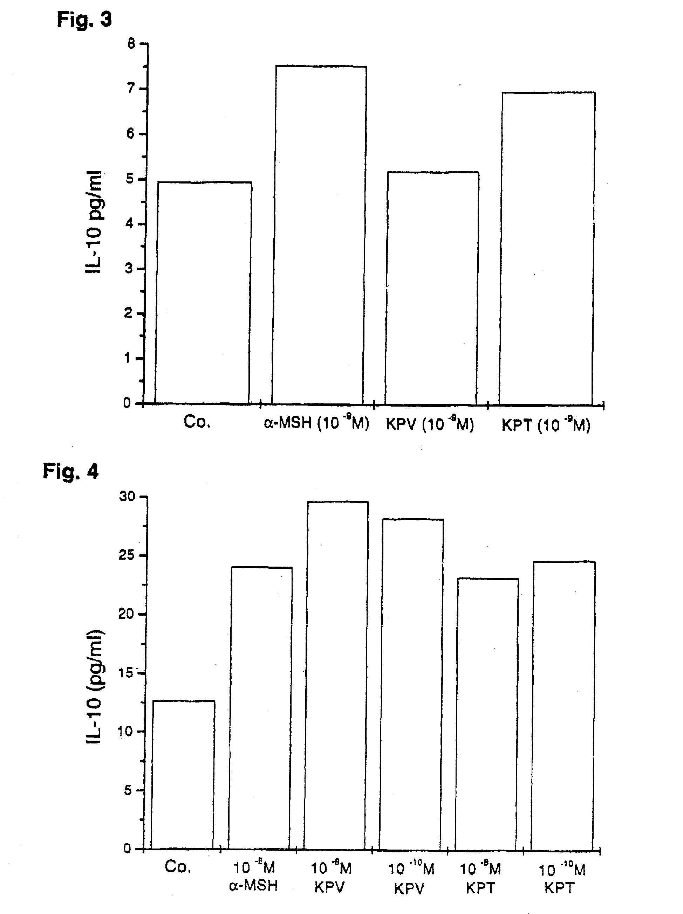

Humane PBMC, die nicht behandelt wurden oder mit verschiedenen Konzentrationen an αMSH oder Peptiden behandelt worden sind, produzierten nach 24 Stunden Inkubation nur niedrige Konzentrationen an IL-10 (5-10 pg/ml). αMSH (10⁻¹¹ M), KPV (10⁻⁸ bis 10⁻⁹ M) und KPT (10⁻⁸ bis 10⁻⁹ M) induzierten offensichtlich IL-10-Produktion (siehe Figur 3).

Nach 48 Stunden Inkubation produzierten die humanen PBMC signifikante Mengen an IL-10. αMSH, KPV und KPT erhöhten signifikant die Produktion von IL-10 durch humane PBLC. Es gab keinen wesentlichen Unterschied zwischen αMSH und den Peptiden (siehe Figur 4).

Die gezeigten Ergebnisse beweisen, daß das Peptid KPT wie αMSH und KPV nach intravenöser Applikation die Sensibilisierung von CHS hemmen kann und Hapten-spezifische Toleranz induzieren kann. KPT ist auch in der Lage, IL-10 in vivo und in vitro zu induzieren. Die Daten machen es auch wahrscheinlich, daß der immunsuppressive Effekt von αMSH in vivo nicht nur von der IL-10-Induktion abhängt.

### Beispiel 3

### Material und Methoden:

Alle Schritte wurden bei 0 bis 4°C durchgeführt. Die monocytische Zellinie THP-1 wurde einmal in PBS, einmal ein saurem Glycinpuffer (50 mM Glycin, 100 mM Natriumchlorid, pH 3) und dreimal mit RPMI gewaschen. Anschließend wurden die Zellen (2,5 x 10⁶ pro ml) in 100 µl RPMI/1% BSA resuspendiert und in 96-Well-Mikrotiterplatten transferiert. Nach Zugabe von Biotin-markiertem αMSH (10⁻¹⁰ M) wurden die Zellen 1 Stunde bei 4°C inkubiert, einmal mit PBS gewaschen, in 100 µl PBS/1% BSA resuspendiert und mit FITCmarkiertem Streptavidin (40 µg/ml) 30 Minuten bei 4°C im Dunklen inkubiert. Nach einem letzten Waschschritt wurden die Zellen in PBS resuspendiert. Die Menge an gebundenem Biotin-markiertem αMSH wurde mit einem Durchflußcytometer analysiert. In Kontrollexperimenten wurden die Zellen ohne Biotin-markiertes αMSH inkubiert aber in der Gegenwart von FITC-Streptavidin. Tote Zellen wurden durch Zugabe von Propidiumiodid kurz vor der FACS-Analyse ausgeschlossen. Die Spezifität der Bindung des Biotinmarkierten MSH wurde durch Zugabe von unmarkiertem αMSH (10⁻⁶ bis 10⁻¹² M) oder KPV oder KPT (10⁻⁶ bis 10⁻¹² M) bestimmt.

### Ergebnisse:

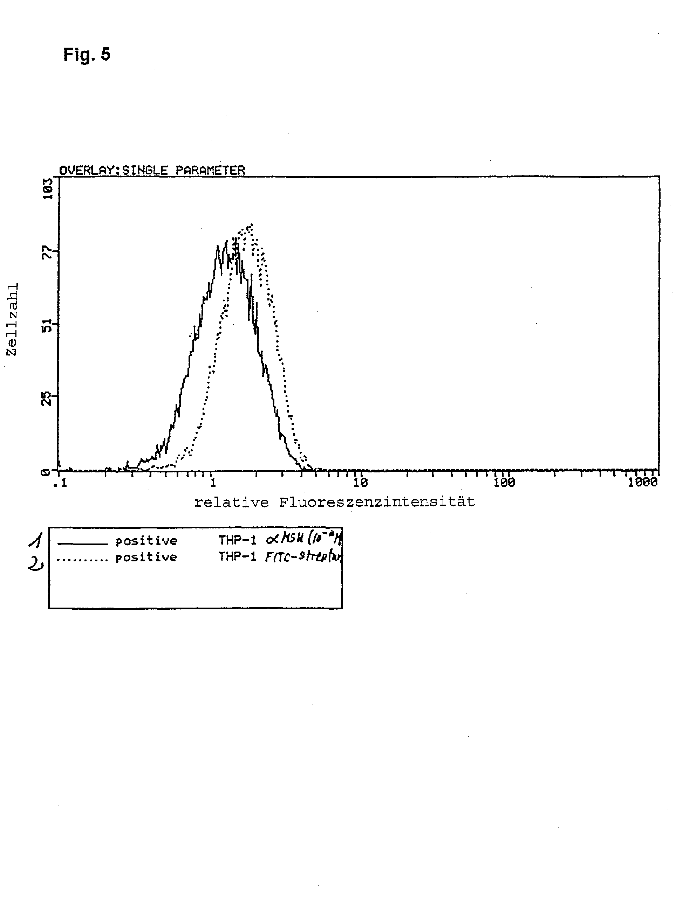

Nach FACS-Analyse mit Biotin-markiertem αMSH exprimieren unstimulierte THP-1-Zellen signifikante Mengen an Bindestellen, die spezifisch sind für αMSH im Vergleich zu Kontrollansätzen, die nur mit FITC-Streptavidin inkubiert werden. In diesem Experiment wurde αMSH in einer Konzentration von 10⁻¹⁰ M eingesetzt (siehe Figur 5).

Um zu bestimmen, ob THP-1-Zellen einen der bekannten Melanocortinrezeptoren (MC) exprimieren, wurde RT-PCR durchgeführt mit MC-1-, MC-2-, MC-3- und MC-4-spezifischen Primern. Gesamt-RNA wurde aus THP-1-Zellen gewonnen. Ein PCR-Produkt spezifisch für MC-1 mit einer erwarteten Länge von 416 bp wurde detektiert (Rajora et al., 1996, J. Leuk. Biol., 59, 248). PCR-Produkte spezifisch für MC-2, MC-3 oder MC-4 wurden nicht nachgewiesen. Die Ergebnisse zeigen, daß THP-1-Zellen MC-1 exprimieren, welches im Gegensatz zu anderen Melanocortinrezeptoren spezifisch für αMSH und ACTH ist.

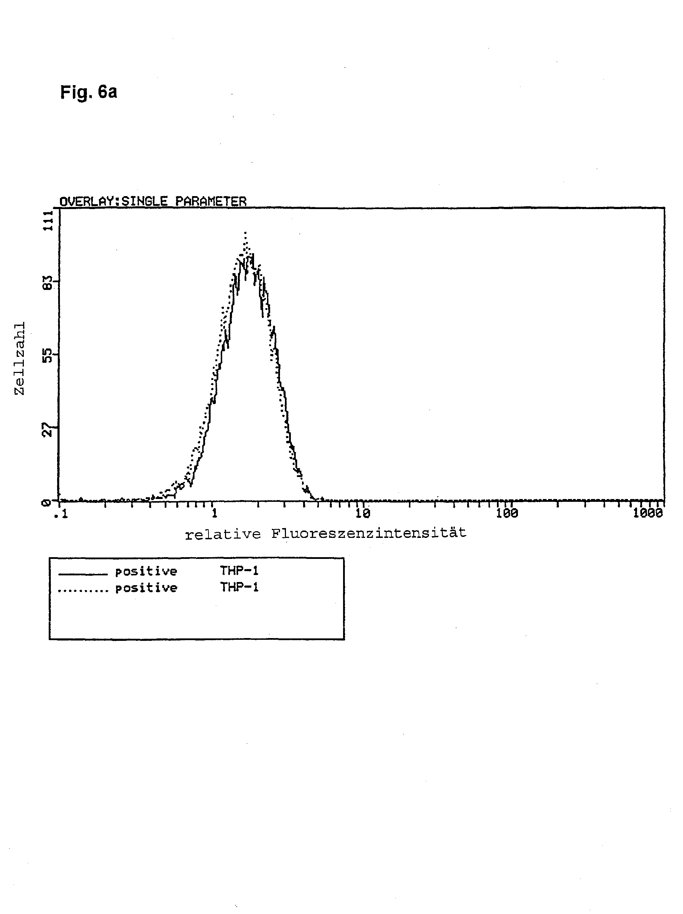

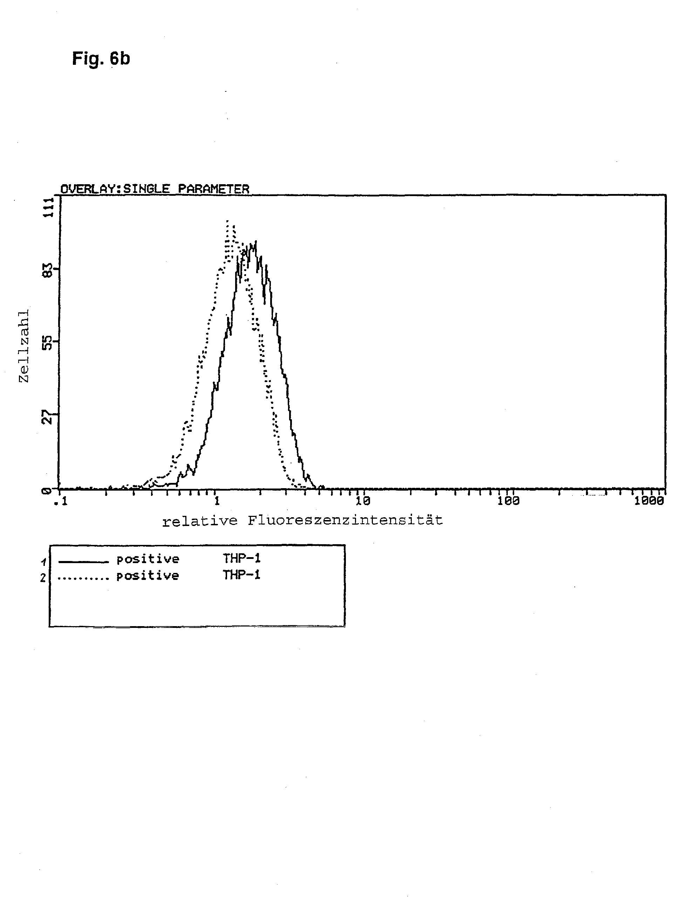

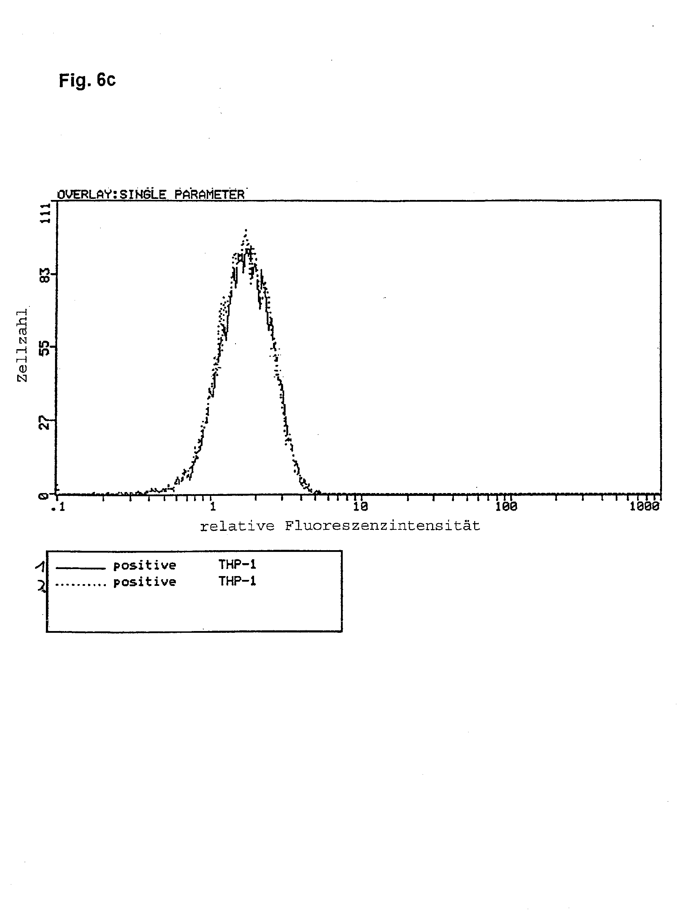

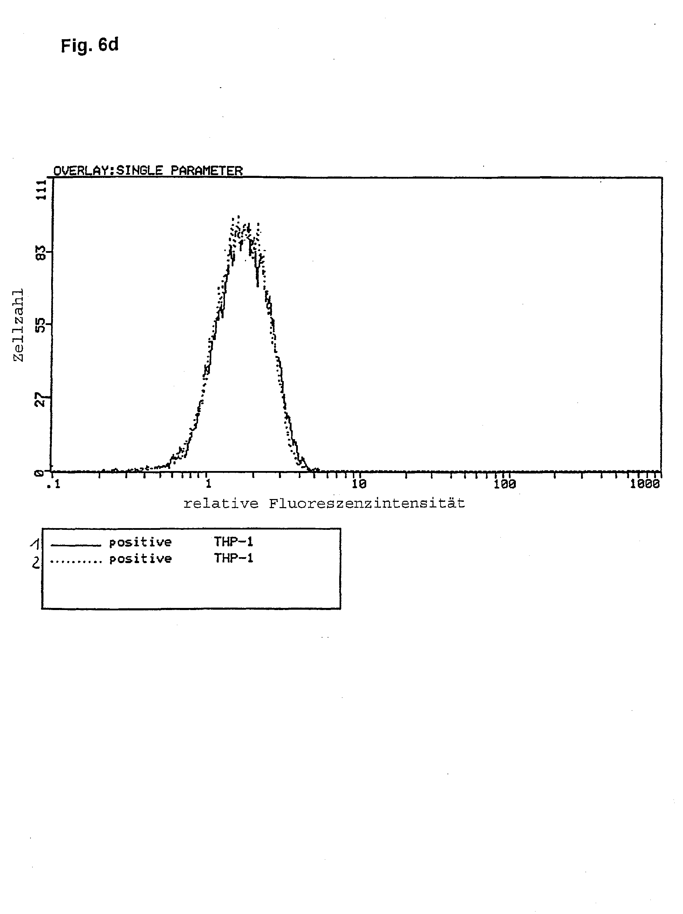

Um zu untersuchen, ob die Bindestellen, die auf THP-1 exprimiert werden, spezifisch für αMSH sind, wurden Kompetitionsexperimente mit αMSH oder KPV oder KPT durchgeführt. Die spezifische Bindung wurde ermittelt durch Inkubation von THP-1-Zellen mit Biotin-markiertem αMSH (10⁻¹⁰ M) und unterschiedlichen Konzentrationen an unmarkiertem αMSH oder Peptiden. Unmarkiertes αMSH in einer Konzentration von 10⁻⁸ M unterdrückte signifikant die αMSH-Bindung. Wenn αMSH in Konzentrationen von 10⁻⁶ M, 10⁻¹⁰ M oder 10⁻¹² M eingesetzt wurde, konnte keine signifikante Suppression beobachtet werden (Figuren 6a bis 6d).

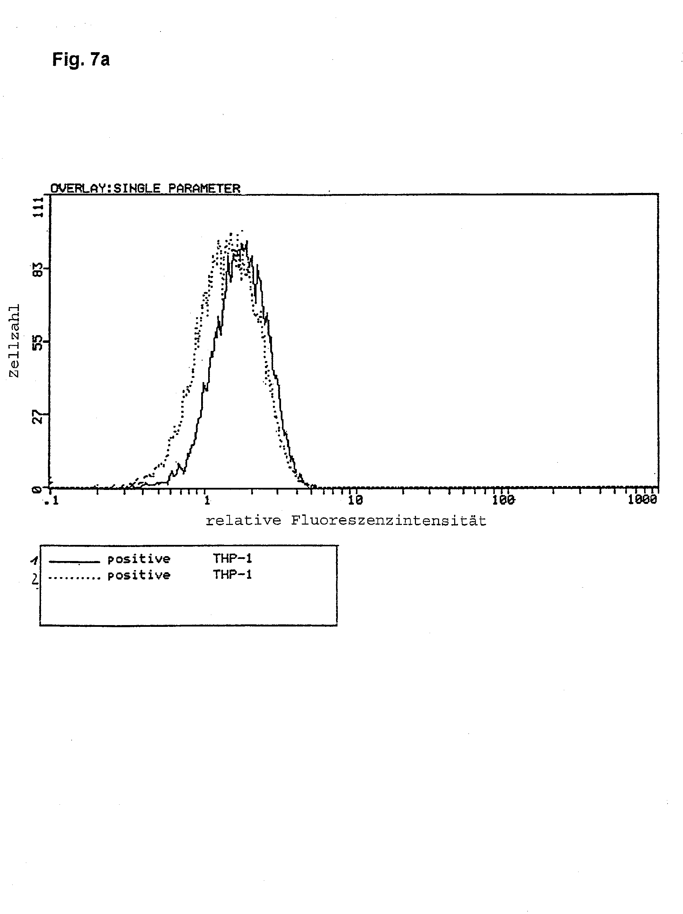

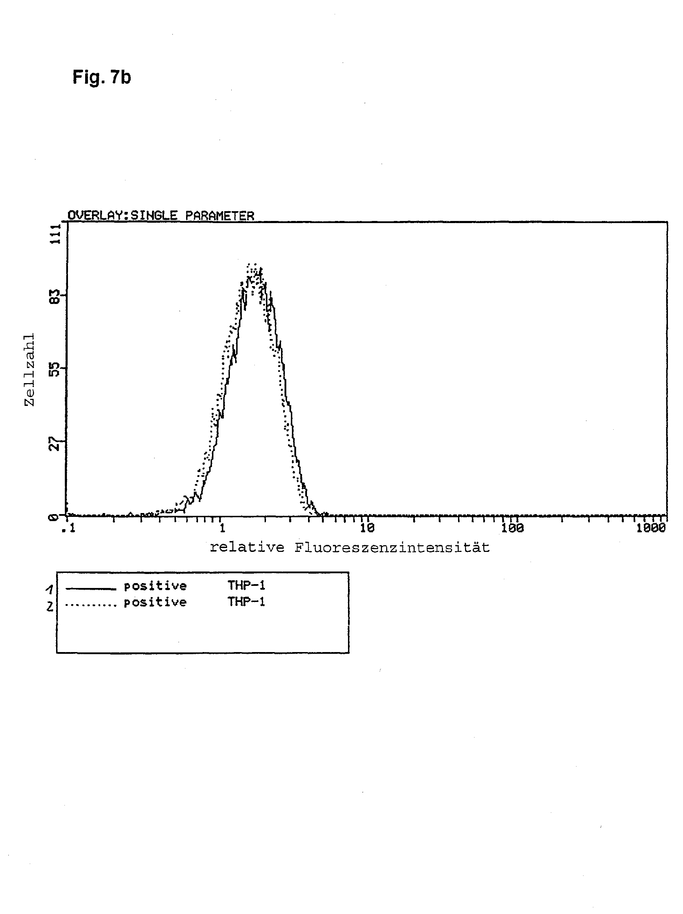

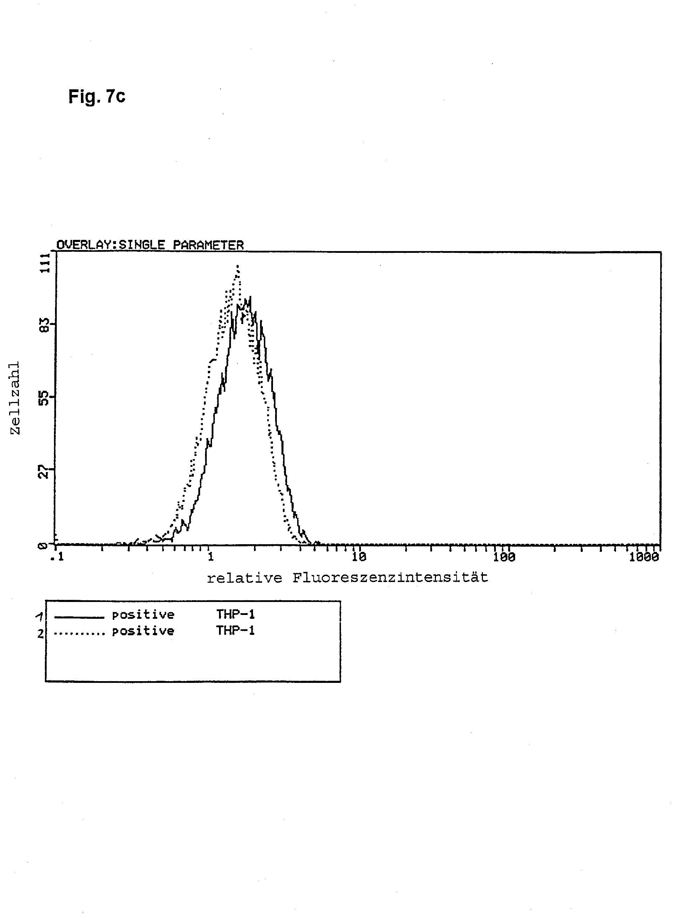

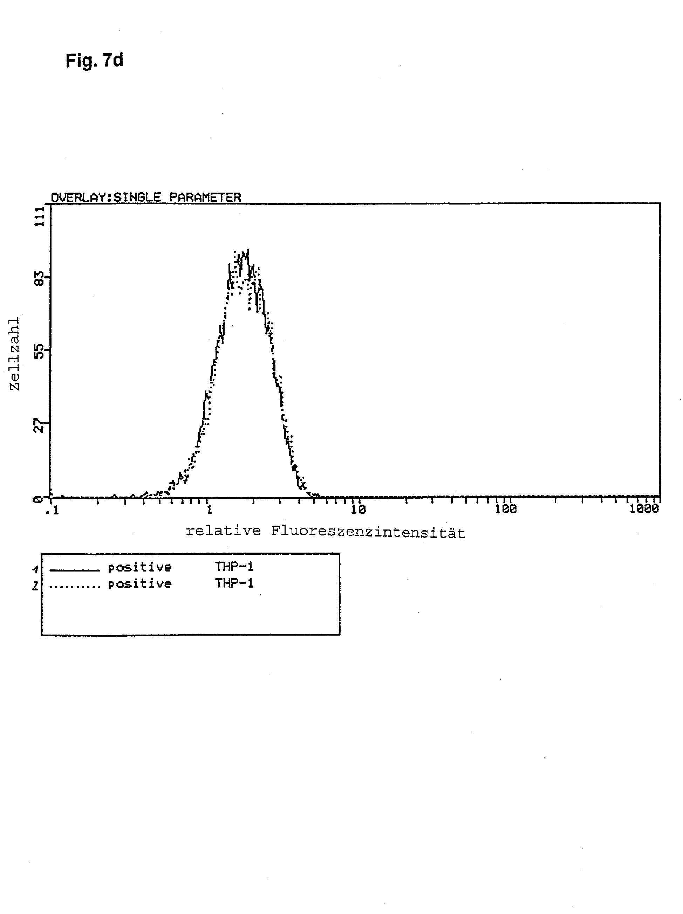

Wenn unmarkiertes KPV eingesetzt wurde, konnte eine signifikante Inhibition nur bei einer Konzentration von 10⁻⁶ M beobachtet werden (siehe Figuren 7a bis 7d).

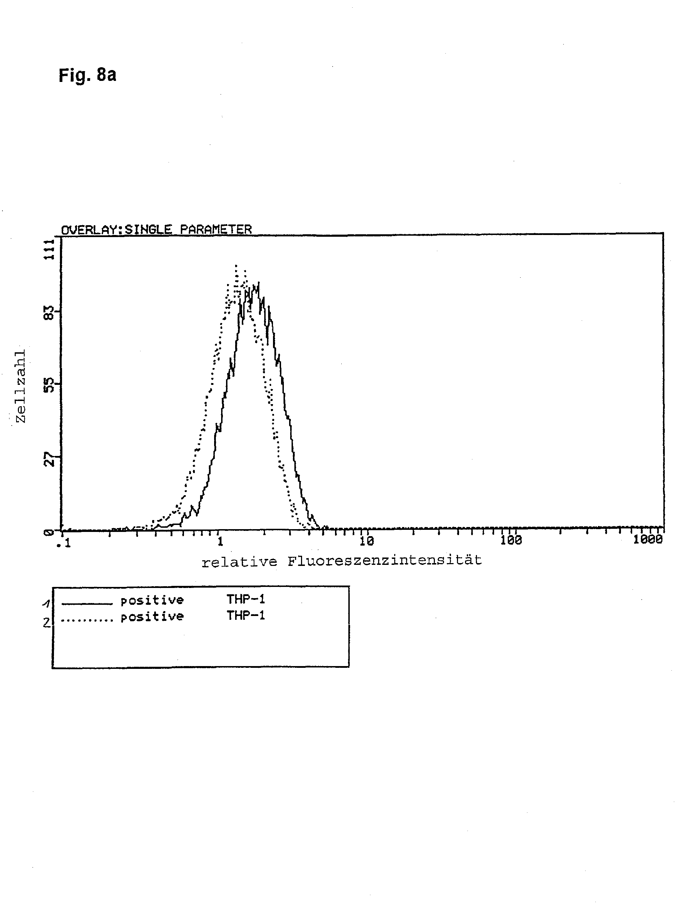

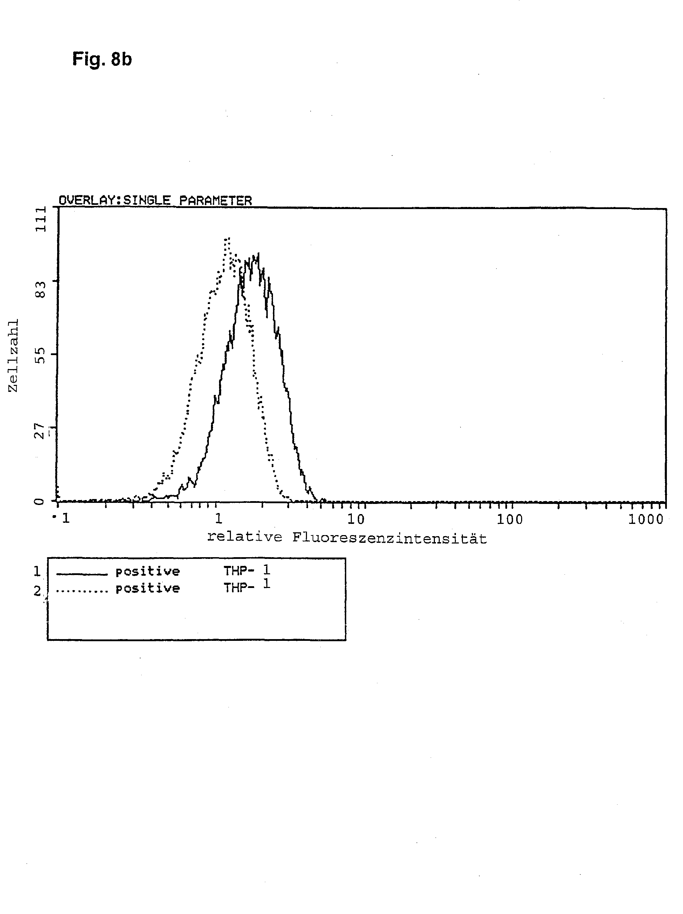

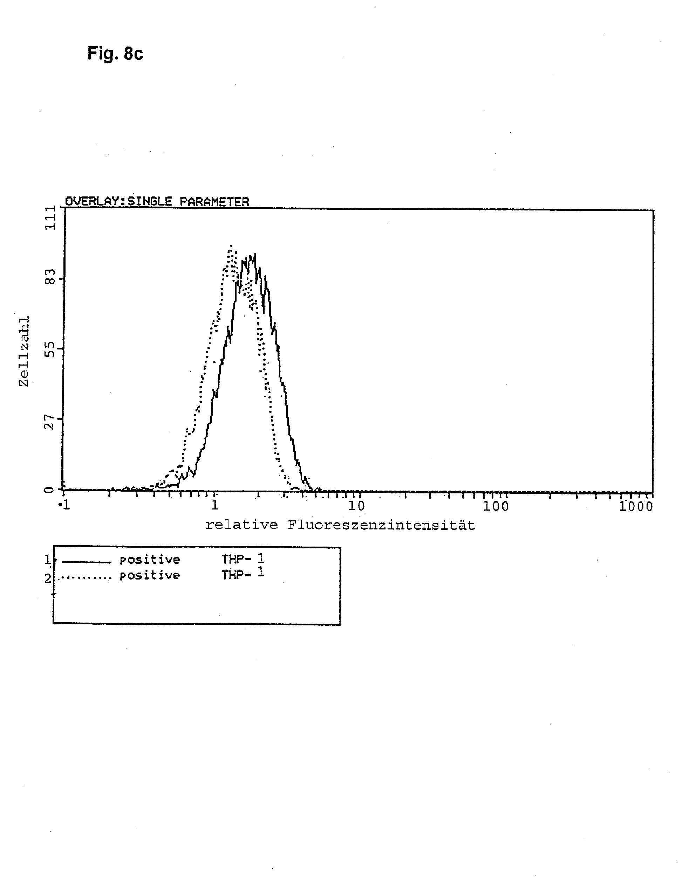

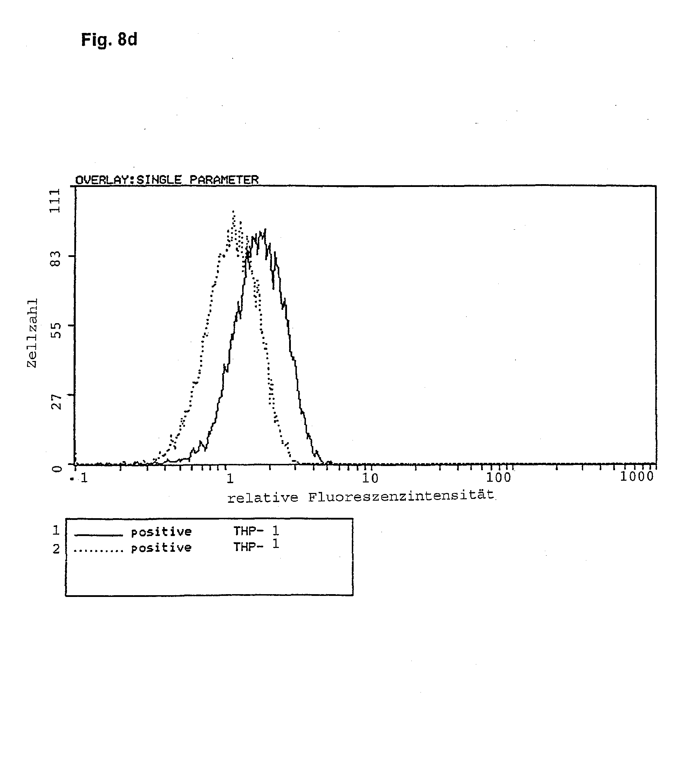

Im Fall des Peptids KPT konnte eine signifikante Hemmung der αMSH-Bindung bei jeder der getesteten Konzentrationen beobachtet werden (10⁻⁶ bis 10⁻¹² M, siehe Figuren 8a bis 8d).

Diese Ergebnisse zeigen, daß das Peptid KPT an den Melanocortinrezeptor auf THP-1-Zellen bindet, der spezifisch für αMSH ist, was darauf hindeutet, daß αMSH und KPT eine gemeinsame Bindestelle aufweisen. Da KPT aber bereits in sehr geringen Konzentrationen Kompetition um den Rezeptor zeigt, ist es wahrscheinlich, daß dieses Peptid sogar eine höhere Affinität zu MC-1-Rezeptor hat als MSH.

### Beispiel 4

### Material und Methoden:

"Human Dermal Microvascular Endothelial Cells" (HDMEC) und die Zellinie HMEC-1 (Human Microvascular Endothelial Cell Line 1) wurden entweder mit TNFα oder LPS in Gegenwart oder Abwesenheit eines der Peptide behandelt. Die Zellen wurden zur RNA-Isolation nach 3 und nach 6 Stunden nach Behandlung gesammelt oder entweder für Adhäsionsmolekül-EIA oder FACS-Analyse 3, 6, 16 oder 24 Stunden nach Behandlung gesammelt. RNA wurde revers transkribiert und Proben einer PCR für E-Selektin, ICAM-1, VCAM oder für β-Aktin als "Housekeeping Gene" unterworfen um eine semiquantitative Bestimmung durchzuführen. Für den Lymphocytenadhäsionsassay wurden die Endothelzellen in Schalen ausgesät und mit ⁵¹Cr-markierten Lymphocyten inkubiert. Nach einem Waschschritt wurde die Menge übriger Lymphocyten, die an den EC-Layer bebunden waren, durch Messung der Radioaktivität in den Proben bestimmt.

### Ergebnisse:

Behandlung der Endothelzellen mit αMSH oder KPT hemmte die LPS- oder TNFαinduzierte Expression von Adhäsionsmolekülen. Dieser Effekt wurde in einem Konzentrationsbereich von 10⁻⁶ bis 10⁻¹² M αMSH oder Peptid beobachtet. Das Peptid KPT hatte den stärksten Effekt auf die Expression von Adhäsionsmolekül-mRNA.

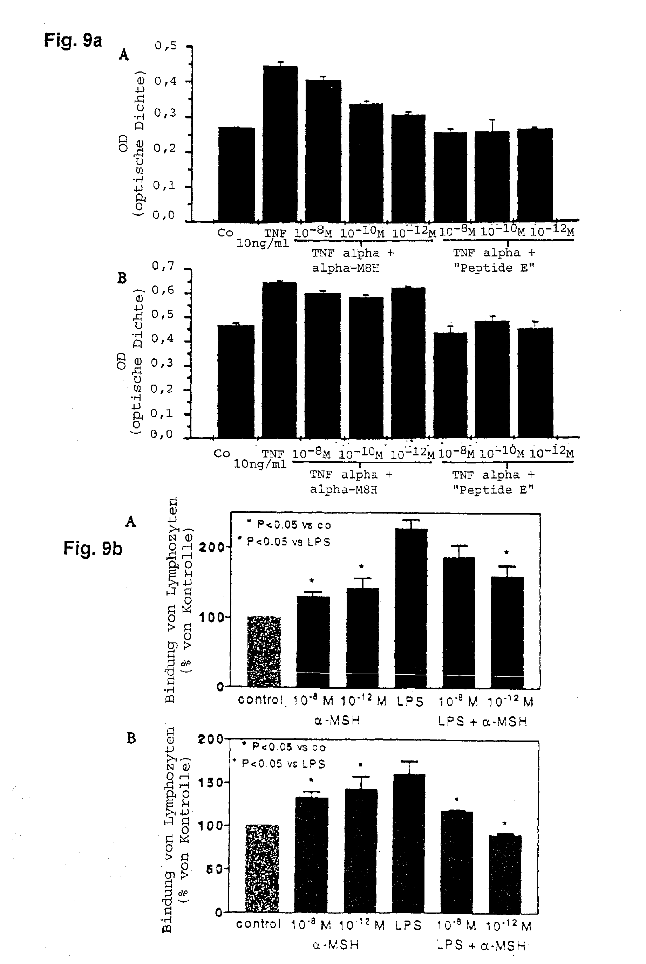

Die durch LPS oder TNFα induzierte Oberflächenexpression von Adhäsionsmolekülen wurde in geringem Maß durch alle Agonisten reduziert. Diese Daten wurden sowohl durch EIA gewonnen, wobei ganze Zellen eingesetzt wurden, als auch durch FACS mit spezifischen Antikörpern (siehe Figur 9a, die EIA-Daten zeigt).

αMSH reduziert signifikant die Bindung von T- und B-Zellen an LPS- oder TNF-αMSHbehandelte EC Layer (siehe Figur 9b). Zusammengenommen zeigen diese Ergebnisse, daß αMSH eine Auswirkung auf die Adhäsion von Lymphocyten an EC hat und somit auch die Extravasation von Lymphocyten in Zuständen der Gewebeentzündung reduziert. Dies wird unterstützt durch die in vivo Daten zur lokalisierten Vaskulitis.

### Beispiel 5

### Material und Methoden:

Epidermale Zellen (ECs) oder normale humane Keratinocyten (HNK) wurden mit IL-1, LPS oder TNFα behandelt in Gegenwart oder Abwesenheit von Peptiden. Nach 15 oder 30 Minuten wurden die Kernproteine gewonnen und einem "Electrophoretic Mobility Shift Assay" (EMSA) mit radioaktiv markiertem Oligonucleotid mit NF-κB-spezifischer Bindungssequenz unterworfen. Unmarkiertes Oligonucleotid wurde als Kompetitor verwendet. In einigen Experimenten wurden Antikörper gegen die p65- oder p50-Kette von NF-κB verwendet, um die Identität der detektierten Banden als entweder p50-Homodimer oder p50/p65-Heterodimer zu bestätigen.

### Ergebnis:

In TNFα- oder LPS-behandelten ECs sowie in IL-1-behandelten HNKs führt die Zugabe der Peptide zu einer reduzierten Aktivierung des Transkriptionsfaktors NF-κB (siehe Figuren 10 und 13a). Dies wiederum führt zu einer Abschwächung der Transkription der Gene für zahlreiche proinflammatorische Mediatoren (Cytokine, Chemokine, Adhäsionsmoleküle, etc.). Die Identität der beobachteten Banden in dem EMSA als NF-κB-Heterodimer wurde durch Einsatz von Anti-p65-Antikörper bestätigt.

### Beispiel 6

### Material und Methoden:

Mäuse wurden durch s.c.-Injektion an einem Ohr mit LPS behandelt. Diese vorbereitende Injektion induziert einen lang andauernden Anstieg in der E-Selektinexpression an der Stelle der LPS-Injektion. 24 Stunden später wurde eine zweite LPS-Dosis i.p. injiziert (Challenge). Diese zweite LPS-Injektion führt zu schneller Gefäßnekrolyse und zur Bildung petechialer Läsionen, die aufgrund ihrer Größe und Anzahl leicht ermittelt werden können. αMSH (25 µg) wurde zum Zeitpunkt der vorbereitenden LPS-Injektion verabreicht.

### Ergebnis:

Injektion von αMSH zum Zeitpunkt der vorbereitenden LPS-Verabreichung hemmt die Induktion lokaler E-Selektinexpression im Ohr (siehe Figur 11a) und reduziert signifikant die Anzahl und Größe der petechialen Läsionen, die nach der "Challenge"-Injektion von LPS gebildet werden (siehe Figur 11 b).

### Beispiel 7

### Material und Methoden:

Dendritische Zellen aus Knochenmark (BMDC) wurden aus den Femurknochen von Mäusen isoliert und mit IL-4 und GM-CSF für 6 oder 9 Tage behandelt. An Tag 6 bzw. 9 wurden die Zellen mit αMSH (2 x 10⁻¹¹ M) oder dem Peptid KP (2 x 10⁻⁶ M) 3 Stunden und 2,5 Stunden vor Reinjektion in naive Mäuse mit demselben genetischen Hintergrund behandelt. 2 Stunden vor Reinjektion wurden die Zellen mit Hapten (1 mM DNBS, der wasserlöslichen Form von DNFB) behandelt. Unmittelbar vor der Reinjektion wurden die Zellen 2 x mit PBS gewaschen. 5 x 10⁻⁵ Zellen wurden je Tier i.v. injiziert. Kontrollzellen wurden entweder mit DNBS allein oder αMSH allein behandelt oder wurden unbehandelt gelassen. 5 Tage nach Injektion wurden die Tiere am Ohr mit DNFB kontaktiert und die Ohrdicke wurde am nächsten Tag gemessen. 2 Wochen später wurden die Tiere mit DNFB resensibilisiert und wiederum 5 Tage später am Ohr rekontaktiert. Schließlich wurde die Ohrschwellung gemessen.

### Ergebnisse:

Empfängertiere, die mit unbehandelten Zellen oder αMSH-behandelten Zellen injiziert wurden, zeigten keine Immunreaktion nach der ersten "Challenge", wie erwartet. Empfängertiere, die mit DNBS-behandelten BMDC injiziert worden waren, zeigten eine entsprechende CHS-Reaktion zum Zeitpunkt der ersten "Challenge". Diese Reaktion wurde in Tieren suprimiert, die mit Zellen injiziert worden waren, die vorher mit TNBS und αMSH oder TNBS und KP in vitro behandelt worden waren. Somit ist der Kontakt von DCs mit αMSH oder Peptid hinreichend um die Hemmung der CHS auszulösen (siehe Figur 12).

Zum Zeitpunkt der zweiten "Challenge" und der entsprechenden Resensibilisierung zeigten Tiere, die mit DNBS behandelten Zellen wiederum injiziert worden waren, keine Immunantwort, wohingegen Tiere, die mit DNBS/αMSH-behandelten Zellen injiziert worden waren, keine Immunantwort zeigten, was darauf hinweist, daß die αMSH-induzierte Toleranz ebenfalls durch DC vermittelt wird (siehe Figur 12).

### Beispiel 8

### "Immuntherapie mit αMSH bzw. αMSH-Derivat-behandelten dendritischen Zellen bzw. T-Zellen"

Dendritische Zellen (DC) wurden isoliert (aus Blut, Knochenmark oder Gewebe). Es können aber auch Zellgemische, die DC enthalten verwendet werden (z.B. epidermale Zellgemische) und in Gegenwart von GM-CSF und IL-4 (bevorzugt: 250-1000 µ/ml für jede der Substanzen) kultiviert.

Nach einer Reifungszeit (bevorzugt 6-9 Tage) werden die Zellen mit Antigen beladen (Konzentration hängt ab vom jeweiligen Antigen, dito Zeitraum) und mit αMSH oder Derivaten davon behandelt. Die Derivate entsprechen zumindest den Aminosäuren 12 und 13 des αMSH (Lys-Pro), bevorzugt sind Lys-Pro-Val enthaltende Derivate. D- und L-Konfiguration der AS sind möglich, dito konservative AS-Austausche. Dies führt u.a. dazu, daß auch das vom IL-1β abgeleitete Lys-Pro-Thr verwendet werden kann, sowie Derivate hiervon die N-terminal verlängert sind. Auch C-terminal verlängerte Derivate können eingesetzt werden. Der Zusatz des Peptids kann vor dem Zusatz des Antigens erfolgen, gleichzeitig, später, einfach oder mehrfach (bevorzugte Dosis ist abhängig vom jeweiligen Peptid, für αMSH z.B. 10⁻⁸ M bis 10⁻¹⁴ M).

Die so behandelten Zellen werden dann in den Empfängerorganismus i.v. injiziert (i.p. oder s.c. wären auch möglich); Maus: 2 x 10⁵ Zellen etwa untere Grenze. Je nach Antigen ist es dabei ausreichend eine einmalige Injektion oder notwendig mehrere Injektionen vorzunehmen. Auch ist es möglich, daß nach längeren Zeiträumen die Injektionen wiederholt werden müssen (hierzu liegen noch keine Daten vor).

Alternativ ist es möglich, DC außerhalb des Körpers mit T-Zellen in Kontakt zu bringen und dann das Gemisch oder die T-Zellen zu injizieren. Hierbei kann die Antigenbeladung der DC vor dem Kontakt mit den T-Zellen oder währenddessen erfolgen. Die T-Zellen können dabei auch von bereits gegen das jeweilige Antigen sensibilisierten Individuen stammen. In der Maus ist die Untergrenze etwa 1 Mio. T-Zellen, bevorzugt sind mehr Zellen (Fig. 14 und 15).

Vorteile einer derartigen Anwendungsweise sind die Vorbeugung gegen jede Art unerwünschter Immunreaktion, die antigen-spezifisch sind und bei denen antigenspezifische Lymphozyten (B- oder T-Zellen) eine pathogenetische Rolle spielen. Hierzu zählen u.a. Allergien, Autoimmunkrankheiten, chronische Entzündungen oder Implantationen. Bei Einsatz genügend hoher Zellzahlen ist auch eine Heilung bereits bestehender Erkrankungen möglich.

Die überraschenden Ergebnisse können ohne an eine Theorie gebunden sein zu wollen darin gesehen werden, daß αMSH ein potenter Immunmodulator ist und zahlreiche antiinflammatorische Eigenschaften besitzt. Hierzu zählt u.a. seine Eigenschaft die Expression co-stimulatorischer Moleküle auf DC zu reduzieren. Ähnliche Eigenschaften weisen auch die erfindungsgemäßen Derivate von αMSH auf, bis hin zum C-terminalen Tripeptid und auch das Dipeptid Lys-Pro. Auch Derivate mit anderer Aminosäurezusammensetzung (konservative AS-Austausche) haben vergleichbare Eigenschaften, hierzu zählt insbesondere das vom IL-1β abgeleitete Lys-Pro-Thr (so daß zu vermuten ist, daß auch N-terminal (analog zu αMSH) verlängerte Peptide mit dem IL-1β co-linearer Sequenz gleich wirken).

In vivo ist αMSH, sowie auch die Derivate, in der Lage hapten-spezifische Toleranz zu induzieren. DC sind professionell Antigen-präsentierende Zellen, die in der Lage sind zahlreiche Typen von Immunreaktionen zu induzieren und die auch den Verlauf derartiger Reaktionen bestimmen. Zu diesen Immunreaktionen zählen vor allem die T-Zell vermittelten Immunreaktionen.

Nun konnte gezeigt werden, daß die in vitro Behandlung von DC oder DC-T-Zellgemischen mit einem Antigen in Gegenwart von αMSH oder Derivaten dazu führt, daß diese Zellen nach Injektion in einen Organismus ebenfalls hapten-spezifische Toleranz induzieren.

Der Mechanismus hier scheint zu sein, daß die Antigenpräsentation der DC durch αMSH oder Derivate so moduliert wird, daß es zur Generierung suppressorischer T-Zellen kommt. So konnte gezeigt werden, daß T-Zellen in entsprechenden Gemischen eine hohe Expression von CTLA-4 aufweisen (Fig. 16). Dies ist eines der Oberflächen moleküle, die suppressorische T-Zellen charakterisieren.

Mit derartig generierten DC oder T-Zellen ist es möglich vorbeugend Autoimmunerkrankungen, chronische Entzündungen oder Allergien zu verhindern. Auch einer Abstoßung von Im- oder Transplantaten kann vorgebeugt werden. Bei Verwendung genügend hoher Zellzahlen ist auch eine Heilung eines bereits bestehenden Erkrankungszustandes denkbar.

Die erfindungsgemäßen Verbindungen können auch zur Tumorbehandlung mittels einer in situ-Aktivierung von dendritischen Zellen verwendet werden. Diese Methode kann möglicherweise auch zur Tolerisierung in Gegenwart der erfindungsgemäßen Peptide herangezogen werden.

Die vorliegende Erfindung betrifft die folgenden Gegenstände (1) bis (18):
(1) Verwendung einer Verbindung der Formel (I) 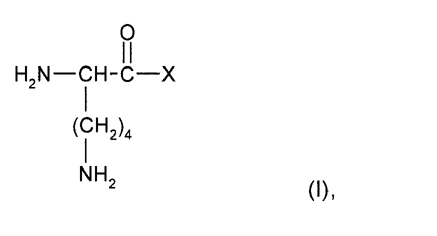 wobei X eine Hydroxylgruppe, eine Aminogruppe, Alkoxy, Pro oder Pro-Thr ist, oder eines pharmazeutisch verträglichen Salzes davon, zur Behandlung und/oder Vorbeugung von entzündlichen Erkrankungen.
(2) Verwendung nach (1), **dadurch gekennzeichnet, daß** die Verbindung der Formel (I) am N-Terminus acyliert und/oder am C-Terminus amidiert oder verestert ist.
(3) Verwendung nach (1) oder (2), **dadurch gekennzeichnet, daß** die Entzündung ausgewählt ist aus der Gruppe bestehend aus Entzündungen der Haut oder von Gefäßen, allergischen Reaktionen, Autoimmunerkrankungen, Fibrosen, Sklerodermien und Transplantatabstoßung.
(4) Verwendung nach (1), (2) oder (3), **dadurch gekennzeichnet, daß** die Entzündung ausgewählt ist aus der Gruppe bestehend aus Psoriasis, atopische Dermatitis, Rhinitis, Kontaktallergien, Asthma und Nahrungsmittelallergien.
(5) Verwendung nach einem (1), (2), (3) oder (4), **dadurch gekennzeichnet, daß** die Entzündung eine Entzündung der Haut ist.
(6) Verwendung nach (1), (2), (3), (4) oder (5), **dadurch gekennzeichnet, daß** die Verbindung der Formel (I) in Form einer Salbe oder Creme verabreicht wird.
(7) Verwendung nach (6), **dadurch gekennzeichnet, daß** die Verbindung der Formel (I) in der Salbe oder Creme in einer Konzentration von 1 µM bis 1 mM enthalten ist.
(8) Verwendung nach (1), (2), (3), (4) oder (5), **dadurch gekennzeichnet, daß** die Verbindung der Formel (I) intraperitoneal, intravenös oder oral verabreicht wird.
(9) Verwendung nach (8), **dadurch gekennzeichnet, daß** 20 µg/kg Körpergewicht bis 10 mg/kg Körpergewicht der Verbindung der Formel (I) verabreicht wird.
(10) Verwendung nach einem (1), (2), (3), (4), (5), (6), (7), (8) oder (9), **dadurch gekennzeichnet, daß** wenigstens 2 verschiedene Verbindungen der Formel (I) eingesetzt werden.
(11) Verwendung einer Verbindung der Formel (I) als Nahrungsmittelzusatz.
(12) Verwendung einer Verbindung der Formel (I) zur Herstellung einer kosmetischen Zusammensetzung.
(13) Verfahren zur in vitro-Herstellung von Zellen, die Toleranz gegen ein Antigen verleihen können, das folgende Schritte umfaßt:
   a) Bereitstellung von Antigen-präsentierenden Zellen,
   b) in Kontakt Bringen der Zellen mit αMSH oder einem biologisch aktiven Derivat oder Fragment davon und
   c) in Kontakt Bringen der Zellen mit dem Antigen,
      wobei die Schritte b) und c) in beliebiger Reihenfolge oder gleichzeitig durchgeführt werden können.
(14) Verfahren nach (13), **dadurch gekennzeichnet, daß** die Antigen-präsentierenden Zellen im wesentlichen dendritische Zellen sind.
(15) Verfahren nach (13), **dadurch gekennzeichnet, daß** die Antigen-präsentierenden Zellen im wesentlichen epidermale Zellen sind.
(16) Verfahren nach (13), (14) oder (15), **dadurch gekennzeichnet, daß** die Zellen in Schritt b) mit einer Verbindung umfassend αMSH, Lys-Pro-Val, Lys-Pro-Thr, Lys-Pro oder Lys in Kontakt gebracht werden.
(17) Verwendung von αMSH oder einem biologisch aktiven Derivat oder Fragment davon zur Herstellung eines Arzneimittels zur Induktion von Toleranz gegen ein Antigen.
(18) Verwendung nach (17), **dadurch gekennzeichnet, daß** das Arzneimittel Zellen enthält, die durch ein Verfahren nach (13), (14), (15) oder (16) erhältlich sind.

## Patentansprüche

1. Verbindung, ausgewählt aus αMSH, biologisch aktiven Derivaten davon und biologisch aktiven Fragmenten davon, zur Induktion von Toleranz gegen ein Antigen.

2. Verbindung nach Anspruch 1, **dadurch gekennzeichnet, daß** das Arzneimittel Zellen enthält, die durch ein Verfahren erhältlich sind, das folgende Schritte umfaßt:
a) Bereitstellung von Antigen-präsentierenden Zellen,
b) in Kontakt Bringen der Zellen mit der Verbindung; und
c) in Kontakt Bringen der Zellen mit dem Antigen,
wobei die Schritte b) und c) in beliebiger Reihenfolge oder gleichzeitig durchgeführt werden können.

3. Verbindung nach Anspruch 1 oder 2, **dadurch gekennzeichnet, daß** die Antigen-präsentierenden Zellen im wesentlichen dendritische Zellen sind.

4. Verbindung nach einem der Ansprüche 1 bis 3, **dadurch gekennzeichnet, daß** die Antigen-präsentierenden Zellen im wesentlichen epidermale Zellen sind.

5. Verbindung nach einem der Ansprüche 1 bis 4, **dadurch gekennzeichnet, daß** die Verbindung Lys-Pro-Val, Lys-Pro-Thr oder Lys-Pro ist.

6. Verbindung nach Anspruch 5, **dadurch gekennzeichnet, daß** die Verbindung Lys-(D)Pro-Thr ist.

7. Verbindung nach einem der Ansprüche 1 bis 6, **dadurch gekennzeichnet, daß** die Verbindung am N-Terminus acyliert und/oder am C-Terminus amidiert oder verestert ist.

8. Verbindung nach einem der Ansprüche 1 bis 7, **dadurch gekennzeichnet, daß** eine Allergie, Autoimmunerkrankung oder Transplantatabstoßung behandelt wird.

9. Verbindung nach einem der vorhergehenden Ansprüche, **dadurch gekennzeichnet, daß** die Allergie ausgewählt ist aus der Gruppe bestehend aus Kontaktallergien, Asthma und Nahrungsmittelallergien.

10. Verbindung nach einem der vorhergehenden Ansprüche, **dadurch gekennzeichnet, daß** die Verbindung in Form einer Salbe oder Creme verabreicht wird.

11. Verbindung nach Anspruch 10, **dadurch kennzeichnet, daß** die Verbindung in der Salbe oder Creme in einer Konzentration von 1 µM bis 1 mM enthalten ist.

12. Verbindung nach einem der Ansprüche 1 bis 11, **dadurch gekennzeichnet, daß** die Verbindung intraperitoneal, intravenös oder oral verabreicht wird.

13. Verbindung nach Anspruch 12, **dadurch gekennzeichnet, daß** 20 µg/kg Körpergewicht bis 10 mg/kg Körpergewicht der Verbindung verabreicht wird.

14. Verbindung nach einem der vorhergehenden Ansprüche, **dadurch gekennzeichnet, daß** wenigstens 2 verschiedene Verbindungen, ausgewählt aus αMSH, biologisch aktiven Derivaten davon und biologisch aktiven Fragmenten davon, eingesetzt werden.
